# EUROPEAN PATENT SPECIFICATION

(11) **EP 3 479 761 B1**
(45) Date of publication and mention of the grant of the patent: **06.09.2023**
(21) Application number: 18204011.3
(22) Date of filing: 02.11.2018
(51) Int. Cl.: A61B 5/0215, A61B 5/20, A61B 5/00, A61B 5/0205

(54) **METHODS AND SYSTEMS FOR DETERMINING DIURETIC RESPONSE PROFILES**
VERFAHREN UND SYSTEME ZUM BESTIMMEN VON DIURETIKA-REAKTIONSPROFILEN
PROCÉDÉS ET SYSTÈMES POUR DÉTERMINER DES PROFILS DE RÉPONSE DIURÉTIQUE

(30) Priority: 03.11.2017 US 201715803580
(43) Date of publication of application: 08.05.2019
(73) Proprietor: PACESETTER, INC., Sylmar, CA 91342-3577 (US)
(72) Inventor: BHARMI, Rupinder, Canyon Country, CA 91387 (US); ADAMSON, Philip B., Austin, TX 78738 (US); AGARWAL, Rahul, San Jose, Santa Clara, CA 95131 (US)
(74) Representative: Barker Brettell Sweden AB

(56) References cited:
- US-A1- 2007 088 220
- US-A1- 2014 155 769
- RUDOLPH W ET AL: "Effectiveness of molsidomine in the long-term treatment of exertional angina pectoris and chronic congestive heart failure", AMERICAN HEART JOURNAL, ELSEVIER, AMSTERDAM, NL, vol. 109, no. 3, 1 March 1985 (1985-03-01) , pages 670-674, XP022950677, ISSN: 0002-8703, DOI: 10.1016/0002-8703(85)90678-7 [retrieved on 1985-03-01]
- STEVENSON L W ET AL: "Effects of afterload reduction (diuretics and vasodilators) on left ventricular volume and mitral regurgitation in severe congestive heart failure secondary to ischemic or idiopathic dilated cardiomyopathy", AMERICAN JOURNAL OF CARDIOLOGY, CAHNERS PUBLISHING CO., NEWTON, MA, US, vol. 60, no. 8, 15 September 1987 (1987-09-15), pages 654-658, XP023207457, ISSN: 0002-9149, DOI: 10.1016/0002-9149(87)90376-6 [retrieved on 1987-09-15]

## Description

### TECHNICAL FIELD

The present disclosure is directed to systems for determining diuretic response profiles. The systems include obtaining pulmonary artery pressure measurements from a patient prior to and after medication has been taken by the patient, comparing the pressure measurements, and then determining the medication response profile in the patient.

### BACKGROUND

Heart failure (HF) is a debilitating, end-stage disease in which abnormal function of the heart leads to inadequate blood flow to fulfill the needs of the body's tissues. Typically, the heart loses propulsive power because the cardiac muscle loses capacity to stretch and contract. Often, the ventricles do not adequately fill with blood between heartbeats and the valves regulating blood flow may develop leaks, allowing regurgitation or backflow of blood. The impairment of arterial circulation deprives vital organs of oxygen and nutrients. Fatigue, weakness, and inability to carry out daily tasks may result.

HF patients are typically managed using several guideline directed medications for the long-term management of patients with chronic heart failure. Medications, such as angiotensin-converting enzyme (ACE) inhibitors, angiotensin receptor blockers (ARBs) and beta-blockers, are known to prolong life and reverse the progression of heart failure. These medications have target dosing recommended in the guidelines that conform to the methods of clinical trials in which the drugs were validated for use.

Diuretics are one of the most often used medications in treating patients with HF. Diuretics are used to relieve excess volume accumulation that is characteristic of HF, and are effective in treating HF by increasing urine output by the kidney, i.e., promote diuresis. This is accomplished by altering how the kidney handles sodium. If the kidney excretes more sodium, then water excretion will also increase. Most diuretics produce diuresis by inhibiting the reabsorption of sodium at different segments of the renal tubular system. Sometimes a combination of two diuretics is given because this can be significantly more effective than either compound alone (synergistic effect). The reason for this is that one nephron segment can compensate for altered sodium reabsorption at another nephron segment; therefore, blocking multiple nephron sites significantly enhances efficacy.

To-date, however, no prospective clinical trial has compared diuretic use with placebo use and, thus, no target dosing is available. As such, diuretics have a level of evidence "C" (expert opinion) for use in heart failure patients, but no means to determine what specific dose is needed for what patient at what time. There is a need, therefore, to closely manage changes in diuretic intake under guidance of diagnostic blood tests for optimal medical management and outcomes.

US 2007/0088220 discloses an implantable medical device receiving a pulmonary artery pressure (PAP) signal indicative of circulatory blood volume and detecting hypovolemia from that PAP signal. Rudolph and Dirschinger, Effectiveness of molsidomine in the long-term treatment of exertional angina pectoris and chronic congestive heart failure, American Heart Journal (1985), 109(3): 670-674, discloses that 7 days of continuous treatment with 4 mg of molsidomine four times a daily lead to comparable reductions in pulmonary artery pressure in patients with chronic, stable angina pectoris.

US 2014/0155769 discloses devices, systems, and methods for assessing, treating, and for developing new treatments for pulmonary arterial hypertension (PAH) using pulmonary artery pressure (PAP) values and/or cardiac output (CO) estimates. This document also shows a curve presenting the evolution of the PAP over a period of months for a patient suffering from pulmonary arterial hypertension (PAH) before and during a treatment with the diuretic flurosemide (Lasix) and the diuretic metolazone.

### SUMMARY

The invention generally relates to a system for determining a diuretic response profile in a patient as defined in the independent claim. Further embodiments of the invention are defined in the dependent claims.

The claimed invention is directed to a system for determining a diuretic response profile in a patient. The system comprises a memory comprising a database and program instructions and at least one processor. The at least one processor is configured, when executing the program instructions to record, in the database, a first pulmonary artery pressure (PAP) measurement obtained from a patient at time t₁. Time t₁ is prior to the patient taking at least one medication. The medication includes at least one diuretic. The at least one processor is also configured to record, in the database, a second PAP measurement obtained from the patient at time t₂. Time t₂ is subsequent to time t₁ and after the patient has taken the medication. The time t₂ is a time equal to a half-life of the at least one diuretic after the patient has taken the at least one diuretic. The at least one processor is further configured to determine a diuretic response profile of the patient based on the first and second PAP measurements.

The foregoing and other aspects, features, details, utilities and advantages of the present disclosure will be apparent from reading the following description and claims, and from reviewing the accompanying drawings.

### BRIEF DESCRIPTION OF THE DRAWINGS

FIG. 1 is an exemplary block diagram of a method for determining a diuretic response profile in a patient.
FIG. 2 is an exemplary block diagram of a method for determining a diuretic response profile in a population.
FIG. 3 is an exemplary embodiment of a mean PAP following administration of a diuretic to a patient in accordance with the present disclosure.
FIG. 4 is an exemplary embodiment of a mean PAP following administration of a diuretic to a patient in accordance with the present disclosure.
FIG. 5 is an exemplary embodiment of PAP trends in patients in accordance with the present disclosure.

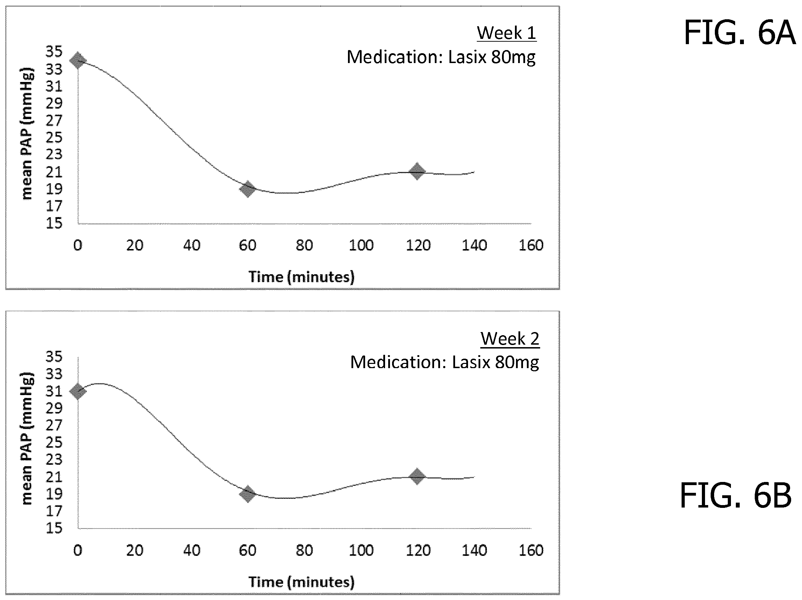

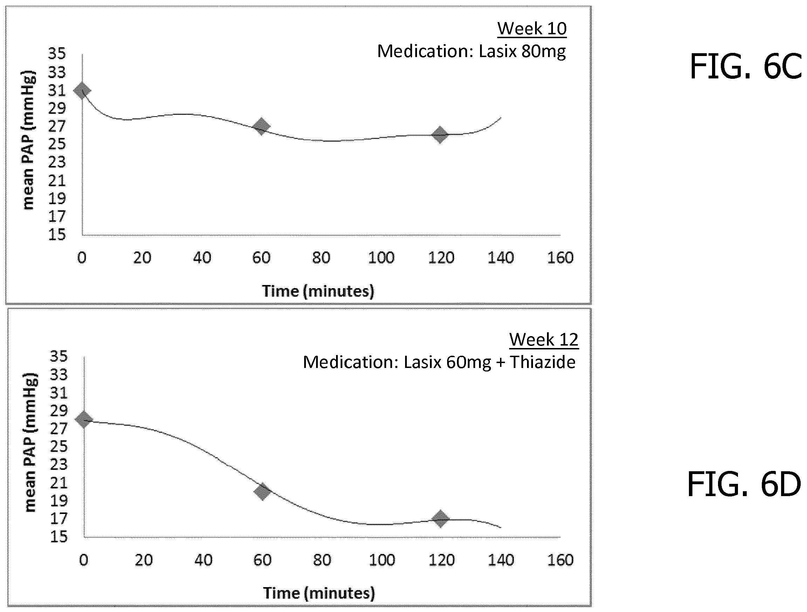

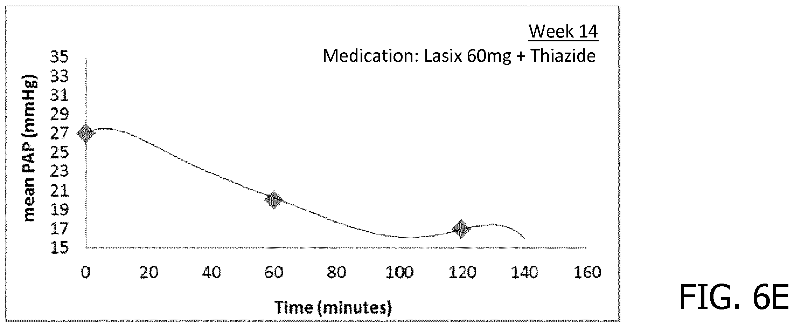

FIGs. 6A-6E are exemplary embodiments of a diuretic response profile in a patient in accordance with the present disclosure. FIG. 6A is an exemplary embodiment of a response profile of a patient after 1 week of medication intake. FIG. 6B is an exemplary embodiment of a response profile of a patient after 2 weeks of medication intake. FIG. 6C is an exemplary embodiment of a response profile of a patient after 10 weeks of medication intake. FIG. 6D is an exemplary embodiment of a response profile of a patient after a medication intake change. FIG. 6E is an exemplary embodiment of a response profile of a patient after 14 weeks of medication intake, including a change of medication at week 12.
FIG. 7 is an exemplary embodiment of a diurnal variation of mean PAP over a population of patients in accordance with the present disclosure.
FIG. 8 is an exemplary embodiment of a system for determining a diuretic response profile in a patient in accordance with the present disclosure.

Corresponding reference characters indicate corresponding parts throughout the several views of the drawings.

### DETAILED DESCRIPTION OF THE DISCLOSURE

While methods do not fall within the scope of the claimed invention, the present disclosure is directed to methods and systems for determining response profiles of patients - both individual patients and those that are part of a population - to a medication, or medications. The methods and systems include obtaining pulmonary artery pressure measurements from a patient prior to and after medication has been taken by the patient, comparing the pressures, and then determining the medication response profile in the patient. The methods and systems may further include modifying the medication, withdrawing the medication, and/or identifying a non-responsive patient.

As used herein, the term "medication" refers to a substance used for medical treatment, such as a medicine or a drug. The term "medication" includes both an individual medicine/drug, or a combination of medicines/drugs. The medications include, for example, a diuretic or a combination of diuretics.

In the context of the claimed invention, the medication comprises at least one diuretic. Diuretics are the corner stone of heart failure (HF) management, and are effective in treating HF by increasing urine output by the kidney, i.e., promote diuresis. This is accomplished by altering how the kidney handles sodium. If the kidney excretes more sodium, then water excretion will also increase. Most diuretics produce diuresis by inhibiting the reabsorption of sodium at different segments of the renal tubular system. Sometimes a combination of two diuretics is given because this can be significantly more effective than either compound alone, i.e., the combination has a synergistic effect. One reason for this is that one nephron segment can compensate for altered sodium reabsorption at another nephron segment; therefore, blocking multiple nephron sites significantly enhances the efficacy of the treatment.

In accordance with the present disclosure, any diuretic known in the art may be used as part of, or as the entire medication. In some embodiments, the at least one diuretic includes at least one of a loop diuretic, a thiazide diuretic, a potassium-sparing diuretic, a mineralocorticoid receptor antagonist, and combinations thereof.

In some embodiments, the loop diuretic includes at least one of torsemide, furosemide, bumetanide, ethacrynic acid, and combinations thereof. In some embodiments, the thiazide diuretic includes at least one of chlorothiazide, chlorothalidone, hydrochlorothiazide, metolazone, indapamide, metolazone, and combinations thereof. In some embodiments, the potassium-sparing diuretic includes at least one of amiloride, spironolactone, triamterene, eplerenone, and combinations thereof. In some embodiments, the mineralocorticoid receptor antagonist includes at least one of eplerenone, spironolactone, canrenone, finerenone, and combinations thereof.

In particular, the present disclosure is directed to providing means of determining a response profile for a patient, e.g., diuretic response profile, and methods of using the response profile to guide the medical management of the patient. Timed measurements of PAPs are taken and are used to derive the diuretic response of an individual patient. The diuretic profile may then be used to determine the bioavailability of the diuretic and used to guide the diuretic dose prescribed for the patient. The profile also provides guidance on which diuretic family to be used on the patient.

Thus, in some embodiments of the present disclosure, a method of determining a diuretic response profile in a patient is disclosed. As already outlined, the method itself does not form part of the claimed invention. The method comprises obtaining a first pulmonary artery pressure (PAP) measurement of a patient at time t₁. Time t₁ is prior to the patient taking at least one medication. The medication includes at least one diuretic. The method also comprises recording the first PAP measurement taken at time t₁, such as in a database. The method further comprises obtaining a second PAP measurement of a patient at time t₂. Time t₂ is subsequent to time t₁ and after the patient has taken the medication. The method additionally comprises recording the second PAP measurement taken at time t₂, such as in the database. The method also comprises determining a diuretic response profile of the patient based on the first and second PAP measurements.

In some embodiments of the present disclosure, the method further includes modifying the medication taken by the patient. Modifying the medication includes, but is not limited to, changing a medication type, adjusting a medication amount, withdrawing the medication, and combinations thereof. Adjusting the medication amount includes either increasing the dosage amount or decreasing the dosage amount. In some embodiments, modifying the medication includes not changing the medication and/or medication amount, but, rather, directing the patient to monitor their diet, e.g., to eat healthier, increase patient compliance in taking the medication, restricting the sodium intake of the patient, and combinations thereof.

In some embodiments, modifying the medication includes combining the at least one diuretic with another diuretic. When a combination of diuretics is used, in some embodiments, the combination creates a synergistic effect on lowering the PAP in a patient. Thus, in some embodiments, the medication includes a synergistic combination of at least two diuretics. In some embodiments, the synergistic combination includes a medication including both thiazide diuretics and loop diuretics.

In some embodiments of the present disclosure, the methods further comprise identifying a non-responsive patient. As used herein, a "non-responsive" patient includes a patient that has taken a particular medication but does not have a positive physical and/or physiological response to that medication, either partially or wholly. That is, the patient may initially have positive physical and/or physiological responses to the medication, but after continued intake of the medication the patient is no longer experience those positive effects. A non-responsive patient also includes a patient who, after taking a particular medication, has no positive physical, functional and/or physiological responses at any time.

In some embodiments, the methods further include determining to use an advanced therapy treatment for a patient. That is, when the diuretic response profile of the patient indicates that a particular medication or medications have not worked and/or are no longer working, a medical personnel member determines to implement an advanced therapy for the patient. As used herein, a medical personnel member includes, but is not limited to, a healthcare professional, a physician, a doctor, and others that are qualified to read and/or record the patient's measurements and make a determination resulting therefrom. The advanced therapy includes, but is not limited to, at least one of dialysis, a ventricular assist device (VAD) implant, a heart transplant, using extracorporeal membrane oxygenation (ECMO), ultrafiltration, inotrope usage, renal replacement, and combinations thereof.

Determining the diuretic response profile includes comparing the first PAP recording to the second PAP recording. Determining the diuretic response profile may include using the comparisons of the first PAP recording and the second PAP recording to determine whether or not to modify the medication. In other embodiments, the method comprises maintaining the medication at its current dosage or dosing regimen. This is often done when improvement in a patient is indicated. That is, if improvement in a patient is indicated after taking a certain medication and/or dosage regimen, then the diuretic response profile determination is to continue to maintain that medication/regimen. In other embodiments, the comparison will lead to the medication being modified or the dosing regimen modified where improvement is not indicated.

When comparing the PAP measurements, determining the response profile includes identifying a change in PAP measurements in the patient and determining an expected change of the PAP measurements in the patient. The expected change includes, but is not limited to, the determination for a specific diuretic, a combination of diuretics, a dosage amount, the physical characteristics of the patient, and combinations thereof.

In some embodiments, determining the response profile includes measuring the bioavailability of at least one diuretic. As used herein, "bioavailability" includes using standard chemical tests known in the art for measuring bioavailability, e.g., dissolution tests. The information gained from the standard testing is used to corroborate the diuretic non-response and is used in conjunction with the response profile to direct the clinical management of the patient. More specifically, bioavailability is a component of the pharmacodynamics and pharmacokinetic evaluation of a drug and is usually a known parameter that is determined by the manufacturer and published as part of phase 1 or 2 investigation. It forms the basis for interval dosing.

As it relates to diuretics, the term "bioavailability" reflects differences in gastrointestinal absorption into the bloodstream, half-life and the recruitment of sodium reabsorption in the distal tubule of the nephron, which reduces the diuretic effect of loop diuretics, e.g., furosemide, torsemide or bumetanide. In some embodiments, the definition of "bioavailability," includes the impact of a specific dose of a specific diuretic on serial measurements of pulmonary artery pressures using an implanted sensor information. This is more "dose effectiveness" which will vary based on the patient, drug and situation.

In accordance with the present disclosure, the timing of the PAP measurements is critical for accurately determining the response profile. That is, the first measurement, taken at time t₁, also referred to as first time or first time point herein, must be before the patient has taken a particular medication. The medication is taken in any known form in the art, e.g., liquid, food, capsule, pill, etc., so long as the medication is taken such that a PAP measurement is taken and recorded.

The second PAP measurement must be taken after the patient has taken the medication at time t₂, also referred to as second time or second time point herein. In a preferred embodiment, time t₂ is after diuresis. In some embodiments, diuresis occurs after diuretic ingestion and results in voiding of at least one fluid from the subject's body. In some embodiments of the present disclosure, time t₂ is from about 30 minutes to about 36 hours after the patient has taken the medication, from about 1 hour to about 12 hours after the patient has taken the medication, or from about 4 hours to about 6 after the patient has taken the medication. In some embodiments, time t₂ is taken at least about 30 minutes, at least about 1 hour, at least about 4 hours, at least about 12 hours, at least about 24 hours, or at least about 48 hours after the patient has taken the medication. For example, in some embodiments, the patient will have a continuous infusion of a diuretic, e.g., furosemide, or a long-term diuretic infusion, e.g., inotrope. In these embodiments, time t₂ is taken after the continuous infusion and/or long-term infusion ceases.

For example, a pre-medication reading of a PAP measurement may be at 30 mmHg, and then after taking furosemide, and activation of its diuretic action, the PAP measurement decreases over a period of about 60 minutes and reaches a steady state at about 20 mmHg. If a medical personnel member were to change the prescription of a patient based on a spot measurement prior to medication ingestion, there may be a danger of over-medication of the patient. Thus, in some embodiments, it is important to adjust the prescription of the current medication dose after diuresis, to avoid, for example, the risk of dehydration which leads to hypotension and renal dysfunction. The lowering of PAP measurements post-medication intake (de-congestive effect) depends on various factors. The present disclosure provides a means of determining the diuretic response for each patient; and provides a method of using this profile to then guide the medical management of the individual patient.

More specifically, in the context of the claimed invention, time t₂ is related to a published half-life of the diuretic ingested by the subject. For example, if a diuretic has a half-life of 6 hours, the measurement is taken at or about 6 hours for time t₂. The objective of time t₂ is to determine if the current diuretic regimen is working, or, if the regimen needs to be modified. That is, time t₂ is used as a guide to help the medical personnel member determine if a dose or drug chance should be made as a result of changes in a diuretic effect.

Further, the methods of the present disclosure are not limited to just two PAP measurements. In particular, in some embodiments, a third, fourth, fifth and sixth PAP measurement are recorded after the patient has taken the medication at time t₃, t₄, t₅ and t₆, respectively. There is no limit to the number of PAP measurements that are taken and recorded. The number of PAP measurements taken and recorded is determined by the choice of the patient's medical personnel member. In some embodiments, the PAP measurements are taken when the patient is asleep. Thus, in some embodiments, the methods disclosed herein comprise obtaining at least two PAP measurements in a patient, wherein the first PAP measurement is taken prior to the patient taking a medication and the second PAP measurement is taken after the patient takes the medication, comparing the PAP measurements, and determining whether to modify the medication.

The measurements taken and recorded from the patient are not limited to PAP measurements. When a PAP measurement is taken, the PAP measurement includes at least one of a mean PAP measurement, a diastolic PAP measurement, a systolic PAP measurement, and combinations thereof. In some embodiments, the PAP measurement includes auxiliary derived metrics from the PAP waveforms. In some embodiments, the metrics include at least one of dP/dt, dicrotic notch pressure, dicrotic notch time, stroke volume, cardiac output, heart rate response, and combinations thereof.

In some embodiments of the present disclosure, determining the diuretic response profile includes deriving the profile while performing a tilt table test to derive an orthostatic response.

In some embodiments of the present disclosure, determining the diuretic response profile includes identifying a diuretic resistance of the medication. As used herein, the term "resistance" refers to congestion refractory to standard diuretic therapy, reduced diuresis and natriuresis upon repeated dosing, and persistent congesting despite increasing daily diuretic doses.

In some embodiments, determining the diuretic response profile occurs at a time after the medication is taken. In some embodiments, determining the diuretic response profile includes predicting a baseline for a patient or population taking the medication. As used herein, the term "baseline" means the stable state of a patient prior to medication ingestion. A post medication time period when the diuretic response profile is determined is during the "dynamic state" of the patient.

In some embodiments, determining the diuretic response profile includes determining a specific dose of the medication to be taken by the patient or a population at a specific time. In some embodiments, the medication includes a dosage amount of from about 1 mg to about 100 mg, from about 5 mg to about 80 mg, from about 20 mg to about 70 mg, or from about 40 mg to about 60 mg of a diuretic or diuretics. It is understood, however, that the dosage amount will vary and is determined based on the discretion of the medical personnel member. That is, the dosage amount of a diuretic, or a combination of diuretics, is according to the standard of care per evidence based medicine as determined by the medical personnel member.

In some embodiments, medications for management of a HF patient are prescribed and changed during clinic visits or during a hospitalization. Diuretic choice and dosages are set based on the patient's current pathology and also with consideration of their lifestyle choices, e.g., high risk behaviors associated with diet, salt intake. The decongestive effect of a diuretic is based on the dose, type of diuretic and bioavailability of, for example, the furosemide/torsemide/bumetanide molecule. For example, in some embodiments, the bioavailability of oral furosemide ranges from about 40 to about 80%, and the bioavailability of torsemide and bumetanide exceed about 80%. Further, in some embodiments, a complication of long-term diuretic therapy is diuretic resistance, which then makes renal function worse. Thus, in some embodiments, giving a patient higher doses results in worse outcomes in a patient.

In some embodiments of the present disclosure, a method of determining a diuretic response profile for a population is disclosed. As used herein, the term "population" includes multiple, i.e., at least two, patients, at least ten patients, at least one hundred patients, at least one thousand patients, or more. The methods and systems used for determining the diuretic response profile for the population include the methods and systems for determining a diuretic response profile in a single patient, as illustrated throughout this disclosure.

In some embodiments, the method comprises obtaining first PAP measurements of a population, wherein the population includes multiple patients, at respective time t₁, wherein respective time t₁ is prior to the patients taking at least one medication. The medication includes at least one diuretic. The method also comprises recording the first PAP measurements taken at respective time t₁, such as in a database. The method further comprises obtaining second PAP measurements of the population at respective time t₂, wherein respective time t₂ is subsequent to respective time t₁ and after the patients have taken the medication. The method additionally comprises recording the second PAP measurements taken at respective time t₂, such as in the database, and determining a diuretic response profile of the population based on the first and second PAP measurements.

In some embodiments of the present disclosure, the population includes a class of patients. The class of patients includes, but is not limited to, at least one of the same or similar sex, gender, race, ethnicity, geographical location, age, disease-type (e.g., chronic kidney disease), physiological disorder, physical traits, co-morbidities (e.g., diabetes, hypertension, atrial fibrillation, etc.), ejection fraction(s), CRT device recipients, valve recipients, body mass index (BMI) and combinations thereof. The class of patients is not limited to the aforementioned groups. Any class of patients is used within the present disclosure so long as the class includes patients with at least one similar or identical characteristic, trait, component, etc.

In some embodiments of the present disclosure, the population includes from about 2 to about 1,000,000 patients, from about 10 to about 100,000 patients, from about 50 to about 1,000 patients, or from about 100 to about 500 patients. The number of patients within the class is not capped, so long as the patients include at least one similar component within the class, e.g., age, sex, etc.

In some embodiments of the present disclosure, each patient in the population takes the same medication. The methods include modifying the medication taken by the population. As discussed throughout this disclosure, modifying the medication includes changing a medication type, adjusting a medication amount, withdrawing the medication, and combinations thereof.

In some embodiments, determining the diuretic response profile includes identifying a change in PAP measurements in the population and determining an expected change in the population. In some embodiments, the expected change is determined for a specific diuretic. In some embodiments, the expected change is identified for a specific class of patients.

In some embodiments, the methods for determining the diuretic response profile in a population include identifying a diuretic response profile of just one individual patient. That is, why obtaining and comparing the data of the population as a whole, when a particular patient begins to show signs of diuretic resistance compared to what the population data indicates, the medication of that one particular patient is modified but that does not necessarily mean the entire population has the medication modified.

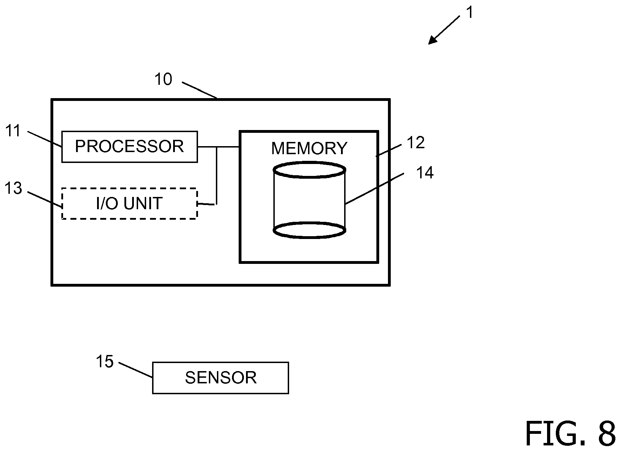

Further to the methods disclosed herein, the present disclosure is also directed to systems for determining a diuretic response profile in a patient, see FIG. 8. The system 1 comprises a memory 12 comprising a database 14 and program instructions, and at least one processor 11. The at least one processor 11 is configured, when executing the instructions, to record, in the database 14, a first PAP measurement is obtained from a patient at time t₁. Time t₁ is prior to the patient taking at least one medication. The medication includes at least one diuretic. The at least one processor 11 is also configured to record, in the database 14, a second PAP measurement obtained from the patient at time t₂. Time t₂ is subsequent to time t₁ and after the patient has taken the medication. The second PAP measurement taken at time t₂ is recorded in the database 14. The at least one processor 11 is further configured to determine a diuretic response profile of the patient based on the first and second PAP measurements.

In some embodiments, the system 1 is used for determining a diuretic response profile in a population. In such embodiments, the at least one processor 11 is configured to record, in the database 14, first PAP measurements obtained from the population at respective time t₁, wherein respective time t₁ is prior to the patients taking at least one medication. The at least one processor 11 is also configured to record, in the database 14, second PAP measurements obtained from the population at respective time t₂, wherein respective time t₂ is subsequent to respective time t₁ and after the patients have taken the medication. The at least one processor 11 is further configured to determine a diuretic response profile of the population based on the first and second PAP measurements.

Similar to the methods disclosed herein, in some embodiments, the systems 1 for determining the diuretic response profile in a patient and for determining a diuretic response profile of a population include the same devices 10, 15, databases 14, and other system components.

In some embodiments, when each PAP measurement is taken, each PAP measurement recording is marked with a unique identifier. Each identifier is then sent to a database. When the identifiers are sent to the database, a data point is generated for each PAP measurement. When the PAP measurements are recorded and sent to the database, a medical personnel member compares the measurements and then determines the diuretic response profile for the particular patient and the particular medication. In some embodiments, the diuretic response profile is determined by creating at least one of a polynomial regression fit, a linear regression, or a non-linear regression of the data points. The linear or non-linear regression includes identifying a time constant of a specific diuretic.

In some embodiments, the system 1 includes a device 15 configured to obtain the first and second PAP measurements in the patient. The device 15 may, for instance, be an implantable wireless sensor 15. The system 1 may then include an input interface 13 configured to receive the PAP measurements from the device 15. In FIG. 15, the input interface 13 is represented by an input/output (I/O) unit 13. The I/O unit 13 may include functions for wired and/or wireless communication, preferably wireless communication, with the implantable wireless sensor 15. In a particular example, the I/O unit 13 may be based on radio circuitry for communication. The I/O unit 13 may be interconnected to the at least one processor 11 and/or memory 12. By way of example, the I/O unit 13 may include any of the following: a receiver, a transmitter, a transceiver, I/O circuitry, input port(s) and/or output port(s).

The at least one processor 11, memory 12 and the I/O unit 13 may be housed in a device 10 that may be implanted in the patient, i.e., in the form of an implantable medical device (IMD), or may be arranged external to the patient, such as worn by the patient in the case of a portable device 10 or arranged elsewhere, for instance, in the patient's home or a medical facility.

In some embodiments, the device 15 is the CARDIOMEMS (Atlanta) heart sensor 15. As described by U.S. Pat. No. 9,265,428 entitled "Implantable Wireless Sensor," these sensors 15 are MEMS-based sensors 15 that are implanted in the pulmonary artery, more particularly in the distal pulmonary artery branch and are configured to be energized with RF energy to return highfrequency, high-fidelity dynamic pressure information from a precisely-selected location within a patient's body.

These sensors 15 are optionally used to generate a real-time or substantially real-time pressure measurement. Via signal acquisition and processing techniques, a pressure measurement is optionally generated via a processor coupled with memory that contains the appropriate algorithm to relate the electrical characteristics of the circuit to the pressure of the pulmonary artery.

In some embodiments, a PAP measurement is obtained with a wireless sensor 15 implanted within a patient. In some embodiments, the sensor 15 is a passive sensor energized to return pressure readings by an electromagnetic field. In addition, one or more of the standard PAP values are optionally determined from a PAP measurement obtained with a wireless sensor 15 implanted in the patient. Therefore, in some embodiments, the PAP measurement is obtained wirelessly and the standard values are determined from a pulmonary artery measurement obtained wirelessly. In either case, the PAP measurement is optionally obtained using an implanted sensor 15. In some embodiments, the implanted sensor 15 is a pressure sensor 15, which, in some embodiments, is implanted in the pulmonary artery of the subject. In some embodiments, the sensor 15 lacks percutaneous connections. In some embodiments, the sensor 15 is energized from an external source. In some embodiments, the sensor 15 is a passive sensor 15 energized to return pressure readings by an electromagnetic field.

In some embodiments, because of the nature of the sensors 15 described above, the PAP measurement is obtained outside of a typical clinical evaluation environment. For example, in some embodiments, the pulmonary artery measurement is obtained while the subject is exercising. Exercising includes any activity of the subject. For example, exercise or exercising includes activities of daily living, prescribed exercise, walking, biking, running or the like. In some embodiments, the PAP measurement is obtained while the subject is asleep.

In some embodiments of the present disclosure, the system includes a network for determining the diuretic response profile of the patient or population. The network is any type of network, including a local area network (LAN) or a wide area network (WAN), or the connection is made to an external computer for example, through the Internet using an Internet Service Provider. One advantage of the use of networks in the present disclosure is that they enable the remote high-volume collection of ambulatory information from the monitoring systems described above. The term "remote" as used herein comprises collection at locations other than hospital locations and in a manner that insubstantially interrupts the daily life activities of the patients. As a result, use of one or more networks enables easy recruitment of larger, more diverse patient populations to be used in development of therapeutics in embodiments of the present disclosure. And, the data collected from those remote locations are more representative of the conditions in which the therapeutics must be safe and effective.

In some embodiments, the system includes a security system. The security system comprises a reverse proxy load balancer with a bit SSL encrypted with purposely limited functionality to protect confidential patient data. With these embodiments, specific ports are opened to specific machines behind the security system, thereby minimizing access to the internal environment. Notably, in some embodiments, all access to a front end computer system, a back end computer system and a physiological information database is through this security system. In some embodiments, other safeguards comprise website timeout after predetermined period of time to prevent unauthorized intrusions from unmonitored workstations. Also, in some embodiments, sensitive patient information is encoded while at-rest within the physiological database. Therefore, even unauthorized access to the database will not provide access to sensitive patient information.

In some embodiments, the systems and methods include a front end computer system, which is configured to act as a depot and gatekeeper to physiological information being communicated from patient monitors through the network before it gets to the database. In particular, the front end computer system is configured to host applications for the consultative addition of correlative information by medical personnel, e.g., a physician. And, primarily, the front end computer system is configured for the accessing and monitoring of data on the individual patient level to enable treatment.

In some embodiments, the front end computer system has functions grouped together, including managing patients, users, thresholds, medical conditions and drugs. In some embodiments, for example and without limitation, various embodiments of the front end computer system are configured to have one or more of the following functions: accept pressure and other physiological data from the patient monitoring systems; process reading data and determine a score based on an automated scoring algorithm; process readings with a passing score further and made available such reading to the appropriate medical personnel; queue readings that do not pass the automated scoring for manual inspection by service providers; use a job queue sub-system to manage the processing of readings and other tasks within the system; provide an interface for medical personnel with appropriate permissions to manage users and site level preferences; provide an interface for medical personnel to import patient data from a thumb drive that was be created as part of the sensor implant procedure; provide an interface for medical personnel to create a patient record substituting for lost patient data; provide an interface for medical personnel to enter and modify patient information; provide an interface for medical personnel to view reading data via trend graphs and individual reading tracings; provide an interface for medical personnel to establish global thresholds for all patients; provide an interface for medical personnel to establish patient specific thresholds; provide an interface for medical personnel to annotate the trend graph with medication changes, notes, and hospitalization events.

In some embodiments, both the service provider and a therapeutic investigator system are connected through a single cloud network. However, it should be appreciated that, in some embodiments, the network comprises a plurality of separate networks. In some embodiments, the service provider is physically resident nearby to the database and the network only a local-area network, while the Internet serves as a longer-distance, more widely accessible network for the medical personnel. Regardless of the structure of the network, it is contemplated that all parties, including the submission, management and retrieval of the information on the physiological information database extend through the security of the security system, which, in these embodiments and without limitation, are a reverse proxy load balancer.

In some embodiments, and as one skilled in the art will appreciate, application software for managing embodiments of the database(s) described herein employ a language such as structured query language (SQL). SQL is a database computer language designed for managing data in relational database management systems (RDBMS), and originally based upon relational algebra. Its scope comprises data insert, query, update and delete, schema creation and modification, and data access control.

In some embodiments, the database(s) employ PostgreSQL, which is an open source object-relational database system particularly well-suited for use on a range of platforms including a Linux-based operating system. It is relatively low-cost, makes for easy development and migrates easily between different operating system platforms. In some embodiments, such software is resident on one or more of the other systems to enable or enhance their ability to interact with the raw data on the database of physiological information.

As will be appreciated by one skilled in the art, aspects of the present disclosure are embodied as a system, method or computer program product. Accordingly, aspects of the present disclosure take the form of an entirely hardware embodiment, an entirely software embodiment including firmware, resident software, micro-code, etc. or an embodiment combining software and hardware aspects that can all generally be referred to herein as a "circuit," "module" or "system." Furthermore, aspects of the present disclosure can take the form of a computer program product embodied in one or more computer readable medium(s) having computer readable program code embodied thereon.

In some embodiments, any combination of one or more computer readable medium(s) are utilized. In some embodiments, the computer readable medium is a computer readable signal medium or a computer readable storage medium. A computer readable storage medium is, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium comprise the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium is any tangible medium that can contain, or store a program for use by or in connection with an instruction execution system, apparatus, or device.

In some embodiments of the present disclosure, a computer readable signal medium comprises a propagated data signal with computer readable program code embodied therein, for example, in baseband or as part of a carrier wave. Such a propagated signal takes any of a variety of forms, including, but not limited to, electro-magnetic, optical, or any suitable combination thereof. A computer readable signal medium is any computer readable medium that is not a computer readable storage medium and that communicates, propagates, or transports a program for use by or in connection with an instruction execution system, apparatus, or device. In some embodiments, the program code embodied on a computer readable medium is transmitted using any appropriate medium, including but not limited to wireless, wireline, optical fiber cable, RF, etc., or any suitable combination of the foregoing.

In some embodiments, computer program code for carrying out operations for aspects of the present disclosure is written in any combination of one or more programming languages, including an object oriented programming language such as Java, Smalltalk, C++ or the like and conventional procedural programming languages, such as the "C" programming language or similar programming languages. The program code executes entirely on the user's computer, partly on the user's computer, as a stand-alone software package, partly on the user's computer and partly on a remote computer or entirely on the remote computer or server. In the latter scenario, the remote computer is connected to the user's computer through any type of network, including a local area network (LAN) or a wide area network (WAN), or the connection is made to an external computer (for example, through the Internet using an Internet Service Provider).

In some embodiments, the computer program instructions are provided to a processor of a general purpose computer, special purpose computer, or other programmable data processing apparatus to produce a machine, such that the instructions, which execute via the processor of the computer or other programmable data processing apparatus, create means for implementing the functions/acts discussed throughout this disclosure.

In some embodiments, the computer program instructions are stored in a computer readable medium that directs a computer, other programmable data processing apparatus, or other devices to function in a particular manner, such that the instructions stored in the computer readable medium produce an article of manufacture including instructions which implement the functions/acts discussed throughout this disclosure.

In some embodiments, the computer program instructions are loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts discussed throughout this disclosure.

Applications described herein are implemented using various software languages, such as Linux. Linux is an open-source software preferred for servers and has the attributes, when applied to embodiments of the present disclosure of agility without sacrificing simplicity, stability or compatibility.

In some embodiments, web or internet applications described herein are implemented using various web programming and application packages, such as Ruby on Rails (RoR). RoR is an open-source web application framework that uses the Ruby programming language. Ruby on Rails comprises tools that make common development tasks easier "out of the box", such as scaffolding that can automatically construct some of the models and views needed for a basic website. RoR, for embodiments of the present disclosure, supplies code efficiency, a relatively short development cycle and it can be run on a JAVA server with Jruby.

### Confidential Information Entered

As the information (PAP measurements, etc.) obtained by the methods and systems of the present disclosure includes physiological information of a patient, it is important to keep this information confidential. Examples of the high-value information that is entered by the medical personnel, associated with the physiological information and then sent for storage on the database include at least one of patient profile and demographic information (age, race, gender, weight, and the like), medical history, medications, classifications, diagnoses, and the like. Other information can comprise at least one of patient episodes, such as surgeries, catheterizations, changes in weight or medication that are associated with a timestamp at entry, and the like. In some embodiments, a front end computer system is configured to associate timestamps for the physiological information with the timestamps of the patient events. In some embodiments, the front end computer system is configured to record information and events that are part of the medical personnel's therapeutic efforts and correlate that information with the physiological information received from the patient monitors.

In some embodiments, the methods and systems of determining diuretic response profiles include the creation and association of files or data that comprise trend graphs selected by the medical personnel. For example, trend lines include trends for systolic, diastolic, mean and pulse pressures, and their respective baselines. In some embodiments, these selected trend lines are associated with start and stop timestamps, and the data is superimposed on the raw physiological data to be stored on the database.

### Exemplary Embodiments

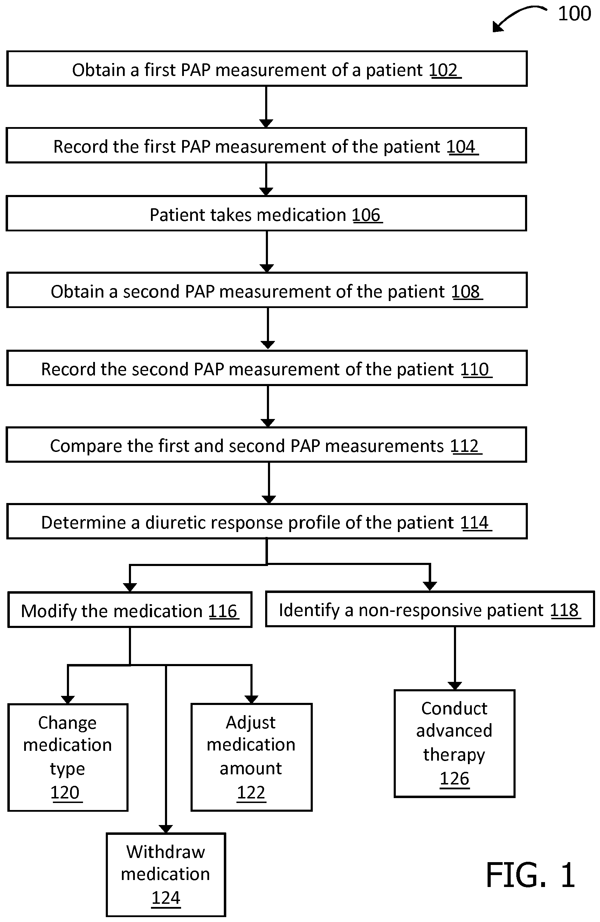

FIG.1 is an exemplary embodiment of a block diagram of a method of determining a diuretic response profile in a patient in accordance with the present disclosure. As already mentioned, the method as such does not form part of the claimed invention.

The method 100 includes obtaining a first PAP measurement of a patient 102. Once the measurement is obtained 102, the first PAP measurement is recorded 104. Steps 102 and 104 occur prior to ingestion of any medication.

After the pre-medication PAP measurement is recorded 104, the patient takes any and all medications 106, including at least one diuretic. Then, the method includes obtaining a second PAP measurement of the patient 108 and recording the second PAP measurement of the patient 110. After both PAP measurements have been recorded, the first and second PAP measurements are compared 112. After the comparison, the diuretic response profile of the patient is determined 114, based on the comparison. For example, if the second PAP measurement is lower than the first measurement, then the medication is performing its intended duty and is working.

If, however, at step 114 the medication is not working and/or producing its intended effect, the medication is modified 116. Modifying the medication 116 includes, but is not limited to, changing the medication type 120, changing the medication amount 122, or withdrawing the medication 124. Once the modification step 116 occurs, the method 100 begins to determine the diuretic response profile of the patient 114.

The method 100 also includes, after determining the response profile of the patient 114, either with or without the medication modification step 116, identifying a non-responsive patient 118. Step 118 occurs when it is determined that the medication is not working and/or not having its intended effect. After a non-responsive patient is identified 118, the method includes conducting and/or recommending advanced therapy 126 on the patient.

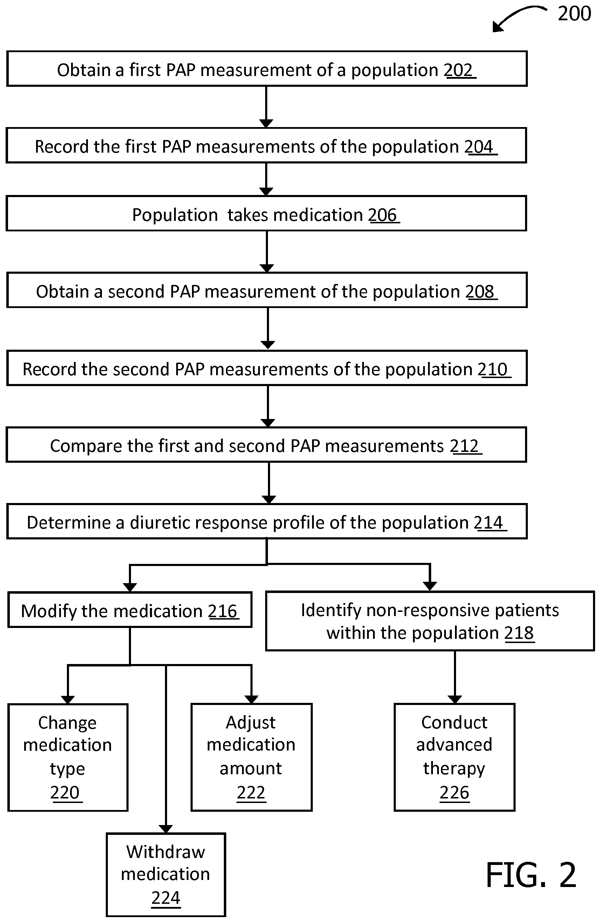

FIG. 2 is an exemplary embodiment of a block diagram of a method of determining a diuretic response profile in a population in accordance with the present disclosure. The method 200 includes obtaining first PAP measurements of a population 202. Once the measurements are obtained 202, the first PAP measurements are recorded 204. Steps 202 and 204 occur prior to ingestion of any medication.

After the pre-medication PAP measurements are recorded 204, the population takes any and all medications 206, including at least one diuretic. Then, the method includes obtaining a second PAP measurement of the population 208 and recording the second PAP measurements of the population 210. After both PAP measurements have been recorded for the population, the first and second PAP measurements are compared 212. After the comparison, the diuretic response profile of the population is determined 214. For example, if the second PAP measurement is lower than the first measurement, then the medication is performing its intended duty and is working for the population.

If, however, at step 214 it is determined that the medication is not working and/or producing its intended effect, then the medication is modified 216. Modifying the medication 216 includes, but is not limited to, changing the medication type 220, changing the medication amount 222, or withdrawing the medication 224. Once the modification step 216 occurs, the method 200 begins again to determine the diuretic response profile of the population 214.

The method 200 also includes, after determining the response profile of the population 214, either with or without the medication modification step 216, identifying a non-responsive patient or patients within the population 218. Step 218 occurs when it is determined that the medication is not working and/or not having its intended effect. After a non-responsive patient or patients are identified 218, the method includes conducting and/or recommending advanced therapy 226 on the patient or patients within the population.

In accordance with the present disclosure, an exemplary embodiment is as follows. A patient implanted with CARDIOMEMS^{™}, instead of simply providing a single reading each day, provides a reading twice a week (or daily) via the following protocol: First, the patient wakes up in the morning and takes a PAP reading prior to any medication ingestion. Immediately thereafter, the patient takes all medications, which include HF medications including diuretic(s). A timer goes off in the bed-side monitor (patient home electronics unit), and about sixty minutes later, the patient takes another PAP recording. As noted elsewhere throughout this disclosure, the timing between readings is variable.

The protocol based recordings are marked with a unique identifier and transmitted to a database (e.g., Merlin.net database). The protocol based PAP recordings are then used to determine the diuretic response profile of the patient. Specifically, the response profile is determined using PAP measurement data-points at time t₁ (pre medication) and time t₂ ("x" minutes after medication) and the time constant of the specific diuretic via, for example, a polynomial regression fit. For example, the time constant of furosemide is shorter than the longer acting thiazide diuretic.

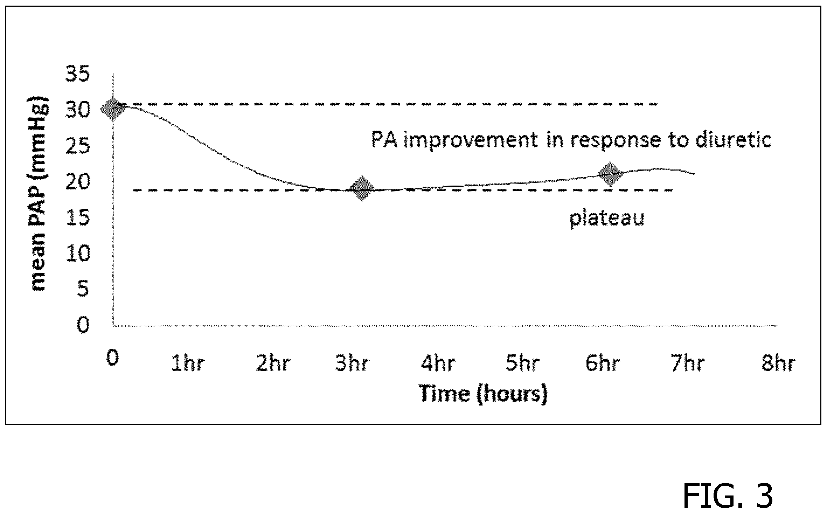

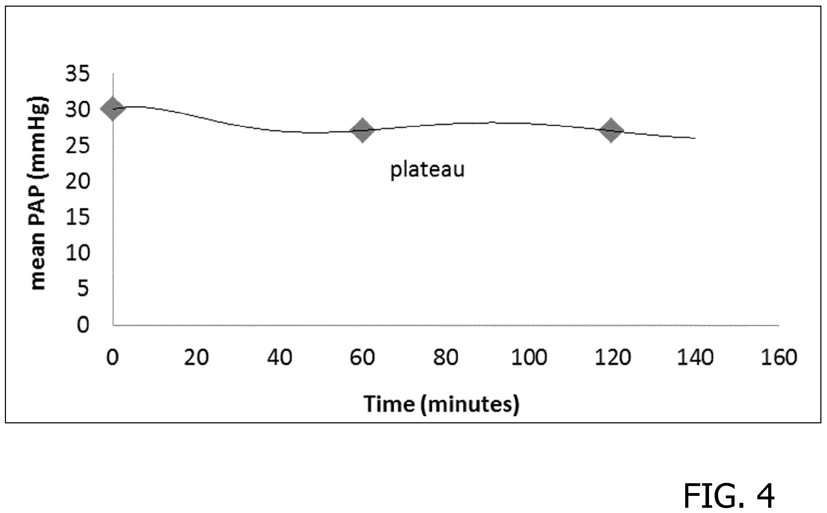

As exemplified in FIGs. 3-4, the lasting effect of the diuretic taken is recorded via the second set of recording data 6-8 hours after the initial recording. FIGs. 3-4 depict an illustration of the mean PAP measurements following administration of a diuretic. As illustrated in FIGs. 3-4, the patient is responding appropriately to the exemplary diuretic, as the PAP measurements decrease over time.

In some embodiments, as an alternative to deriving the diuretic response profile via protocol based recording, the diuretic profile for a population or an individual patient is derived using PAP measurement trends based on incidental recordings. This does not require any special action by a patient, but requires processing of the PAP measurement data wherein recordings that were historically made during a day for a patient are used to determine the aggregated diuretic profile for a patient. In some embodiments, this is also determined for a population.

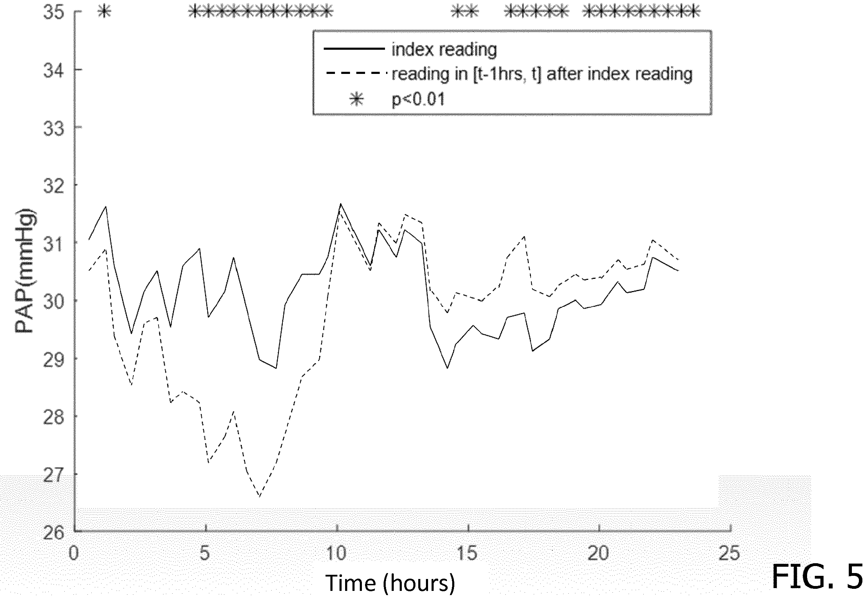

FIG. 5 is an exemplary embodiment of an illustration of a population level diuretic response profile. FIG. 5 depicts the PAP measurement trends following an initial PAP measurement recording each day. If two recordings are incidentally recorded, for example, 6 hours apart, they are used in this analysis to derive the diuretic response profile. On an average for a population, the diuretic medication resulted in reduced PAP measurements, e.g., reducing from 31 ± 11 mmHg to 27 ± 11 mmHg within 6 hours.

### Examples

The following examples describe or illustrate various embodiments of the present disclosure and actually represent comparative examples useful for understanding the invention as defined in the appended claims. Other embodiments within the scope of the appended claims will be apparent to a skilled artisan considering the specification or practice of the disclosure as described herein. It is intended that the specification, together with the examples, be considered exemplary only, with the scope of the invention e being indicated by the claims, which follow the examples.

### Example 1 - Medication Modification

This example examined the characterization or determination of medication dosage and temporal response in a patient given different kinds of medication. Initially, the patient was given a medication of 80 mg of furosemide (Lasix). FIG. 6A shows the patient's PAP measurements at time t₁ prior to medication for one week. The mean PAP measurement of the patient at time t₁ was about 35 mmHg. After the patient took the medication of 80 mg of furosemide and then the second PAP measurement was taken at time t₂, which, as seen in FIG. 6A, is at about 1 hour after medication, the average PAP measurement of the patient was about 19 mmHg, indicating that the medication was working to lower the patient's PAP. The third PAP measurement was taken at time t₃ at about 2 hours after medication. As shown in FIG. 6A, the mean PAP measurement increased slightly to about 21 mmHg, but still showed significant improvement over the pre-medication PAP measurement. In accordance with the present disclosure, a medical personnel member comparing the PAP measurements taken at different times is able to determine that the medication amount (80 mg) and type (furosemide) are working to lower the PAP in the patient, and thus the medication does not necessarily need to be modified.

In the second week of medication, the patient was kept on 80 mg of furosemide. As seen in FIG. 6B, the mean PAP measurement at time t₁ was at about 31 mmHg, which is an improvement over the 35 mmHg mean measurement during week 1. Again, this allows the medical personnel member to determine that the diuretic response profile in the patient is working with the current medication type and dosage amount. As seen in FIG. 6B, the mean PAP measurements at time t₂ and time t₃ during week 2 of the medication are similar to the PAP mean measurements taken during week 1 of the medication at time t₂ and time t₃. Thus, a medical personnel member comparing the PAP measurements is able to determine that the medication amount (80 mg) and type (furosemide) continue to lower the PAP in the patient, and thus the medication does not necessarily need to be modified.

In FIG. 6C, however, the medical personnel member is able to determine that the diuretic profile in the patient is showing early signs of developing diuretic resistance during week 10 of the medication. As shown in FIG. 6C, the profile of the mean PAP measurements at time t₂ and time t₃ began to flatten compared to the mean PAP measurement taken at time t₁ prior to ingestion of the medication. Thus, whereas in FIGs. 6A and 6B the mean PAP measurements were getting lower from about 35 mmHg to about 19 mmHg and from about 31 mmHg to about 19 mmHg, respectively, while still on the same medication type and amount, i.e., 80 mg of furosemide, FIG. 6C shows the patients mean PAP measurements going from about 31 mmHg to only about 27 mmHg. Thus, at this point, the medical personnel member determined that the diuretic profile of the patient was not improving.

As a result, the medical personnel member modified the medication given to the patient. In this example, in week 12, furosemide was still given to the patient, but at a lower dosage amount (60 mg). Additionally, a standard dosage of thiazide was also given to the patient in combination with the furosemide with the goal of lowering the mean PAP measurements. The combination of the furosemide and the thiazide created a synergistic effect on lowering the mean PAP measurements in the patient. That is, as shown in FIG. 6D, the mean PAP measurement during the week 12 medication was about 29 mmHg. After the patient took the combination of 60 mg of furosemide and thiazide, the mean PAP measurement at time t₂ about an hour after the medication was taken was about 19 mmHg and went even lower to about 17 mmHg at time t₃, which was about 2 hours after the medication was taken.

As shown in FIG. 6E, during week 14, the medication remained a combination of 60 mg of furosemide and thiazide and the mean PAP measurements taken at times t₂ and t₃ remained at similar levels (about 19 mmHg and about 17 mmHg, respectively) as the levels at those times during week 12. At this point, the medical personnel member determined that the diuretic profile of the patient had reached an optimal level.

### Example 2 - Diurnal Variation Determination

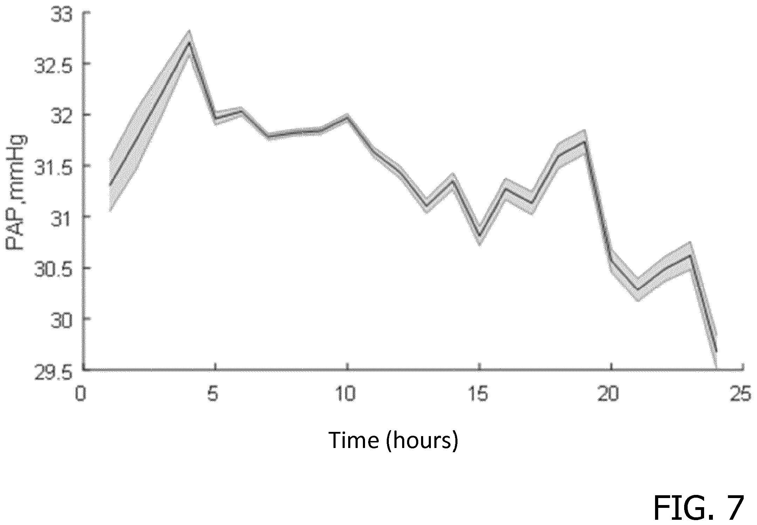

Example 2 discloses a determination of a diurnal variation of mean PAP to better manage a patient. FIG. 7 illustrates the diurnal variation of mean PAP measurements over a population of patients. HF patients generally take two daily doses of their diuretics: a morning dose and an evening dose. As shown in FIG. 7, in accordance with the present disclosure, a medical personnel member is able to determine the diuretic response profile of a patient for both the morning diuretic (dose and time) and the evening diuretic (dose and time), which then allows for the medical personnel member to determine an optimal dosage and time for a patient (either alone or within a population) to take the medication.

For example, as shown in FIG. 7, the mean PAP measurement reached a peak level at about 4:30 a.m. Thus, the medical personnel member determined that the patient take the first dosage at that time. After the medication was taken, the mean PAP measurement level lowered until about 5:00 p.m. and then continued to rise until about 7:30 p.m. Thus, at about 7:30 p.m. the medical personnel member determined that the patient should take the evening dose of the medication and then the mean PAP measurement levels lowered again. Because these levels were tracked and recorded, in the future, a medical personnel member can read the data and determine the response profile in the patient with that particular medication and dosage, and time the medication dosages and types accordingly.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

As various changes could be made in the above constructions without departing from the scope of the disclosure, it is intended that all matter contained in the above description or shown in the accompanying drawings shall be interpreted as illustrative and not in a limiting sense, the invention being defined by the appende claims.

## Claims

1. A system (1) for determining a diuretic response profile of a patient, the system comprises:
a memory (12) comprising a database (14) and program instructions; and
at least one processor (11) configured, when executing the program instructions, to
record, in the database (14), a first pulmonary artery pressure (PAP) measurement obtained from a patient at time t₁, wherein time t₁ is prior to the patient taking the at least one medication;
record, in the database (14), a second PAP measurement obtained from the patient at time t₂, wherein time t₂ is subsequent to time t₁ and after the patient has taken at least one medication and time t₂ is a time equal to a half-life of the at least one diuretic after the patient has taken the at least one diuretic, wherein the at least one medication includes at least one diuretic;
compare the first and second PAP measurements; and
determine a diuretic response profile indicating a temporal response of the patient to the at least one diuretic based on the comparison of the first and second PAP measurements.

2. The system according to claim 1, further comprising a device (15) configured to obtain the first and second PAP measurements in the patient.

3. The system according to claim 2, wherein the device (15) is an implantable wireless sensor (15).

4. The system according to any of claims 1 to 3, wherein the at least one processor (11) is configured, when executing the program instructions, to determine the diuretic response profile comprising at least one of information identifying the patient as a non-responsive patient, information of an advanced therapy treatment for the patient, information of an identified diuretic resistance, and combinations thereof.

5. The system according to any of the claims 1 to 4, wherein the at least one processor (11) is configured, when executing the program instructions, to
record, in the database (14), first PAP measurements obtained from a population of multiple patients at respective time t₁, wherein respective time t₁ is prior to the patients taking the at least one medication;
record, in the database (14), second PAP measurements obtained from the population at respective time t₂, wherein respective time t₂ is subsequent to respective time t₁ and after the patients have taken the at least one medication; and
determine a diuretic response profile of the population based on the first and second PAP measurements.

## Patentansprüche

1. System (1) zum Bestimmen eines Diuretika-Reaktionsprofils eines Patienten, wobei das System folgendes umfasst:
einen Speicher (12), umfassend eine Datenbank (14) und Programmanweisungen; und
mindestens einen Prozessor (11), der, wenn er die Programmanweisungen ausführt, konfiguriert ist zum Aufzeichnen, in der Datenbank (14), einer ersten Messung des Lungenarteriendrucks (PAP), die von einem Patienten zum Zeitpunkt t₁ erhalten wurde, wobei der Zeitpunkt t₁ vor der Einnahme des mindestens einen Medikaments durch den Patienten liegt;
Aufzeichnen, in der Datenbank (14), einer zweiten PAP-Messung, die von dem Patienten zum Zeitpunkt t₂ erhalten wurde, wobei der Zeitpunkt t₂ nach dem Zeitpunkt t₁ liegt und nachdem der Patient mindestens ein Medikament eingenommen hat und der Zeitpunkt t₂ ein Zeitpunkt ist, der gleich einer Halbwertszeit des mindestens einen Diuretikums ist, nachdem der Patient das mindestens eine Diuretikum eingenommen hat, wobei das mindestens eine Medikament mindestens ein Diuretikum beinhaltet;
Vergleichen der ersten und zweiten PAP-Messung; und
Bestimmen eines Diuretika-Reaktionsprofils, das eine zeitliche Reaktion des Patienten auf das mindestens eine Diuretikum angibt, basierend auf dem Vergleich der ersten und zweiten PAP-Messung.

2. System nach Anspruch 1, ferner umfassend eine Vorrichtung (15), die dazu konfiguriert ist, die erste und zweite PAP-Messung bei dem Patienten zu erhalten.

3. System nach Anspruch 2, wobei die Vorrichtung (15) ein implantierbarer drahtloser Sensor (15) ist.

4. System nach einem der Ansprüche 1 bis 3, wobei der mindestens eine Prozessor (11) dazu konfiguriert ist, bei dem Ausführen der Programmanweisungen das Diuretika-Reaktionsprofil zu bestimmen, das mindestens eine von Informationen umfasst, die den Patienten als nicht ansprechenden Patienten identifizieren, Informationen über eine fortgeschrittene Therapiebehandlung für den Patienten, Informationen über eine identifizierte Diuretika-Resistenz und Kombinationen davon.

5. System nach einem der Ansprüche 1 bis 4, wobei der mindestens eine Prozessor (11), wenn er die Programmanweisungen ausführt, konfiguriert ist zum
Aufzeichnen, in der Datenbank (14), von ersten PAP-Messungen, die von einer Population von mehreren Patienten zum entsprechenden Zeitpunkt t₁ erhalten wurden, wobei der entsprechende Zeitpunkt t₁ vor der Einnahme des mindestens einen Medikaments durch die Patienten liegt;
Aufzeichnen, in der Datenbank (14), von zweiten PAP-Messungen, die von der Population zum entsprechenden Zeitpunkt t₂ erhalten wurden, wobei der entsprechende Zeitpunkt t₂ nach dem entsprechenden Zeitpunkt t₁ und nach der Einnahme des mindestens einen Medikaments durch die Patienten liegt; und
Bestimmen eines Diuretika-Reaktionsprofils der Population basierend auf der ersten und zweiten PAP-Messung.

## Revendications

1. Système (1) pour déterminer un profil de réponse diurétique d'un patient, le système comprenant :
une mémoire (12) comprenant une base de données (14) et des instructions de programme ; et
au moins un processeur (11) configuré, lors de l'exécution des instructions de programme, pour
enregistrer, dans la base de données (14), une première mesure de pression artérielle pulmonaire (PAP) obtenue auprès d'un patient à l'instant t₁, dans lequel l'instant t₁ est antérieur à la prise par le patient de l'au moins un médicament ;
enregistrer, dans la base de données (14), une seconde mesure de PAP obtenue auprès du patient à l'instant t₂, dans lequel l'instant t₂ est postérieur à l'instant t₁ et après que le patient a pris au moins un médicament et l'instant t₂ est un instant égal à une demi-vie de l'au moins un diurétique après que le patient a pris l'au moins un diurétique, dans lequel l'au moins un médicament comprend au moins un diurétique ;
comparer les première et seconde mesures de PAP ; et
déterminer un profil de réponse diurétique indiquant une réponse temporelle du patient à l'au moins un diurétique sur la base de la comparaison des première et seconde mesures de PAP.

2. Système selon la revendication 1, comprenant en outre un dispositif (15) configuré pour obtenir les première et seconde mesures de PAP auprès du patient.

3. Système selon la revendication 2, dans lequel le dispositif (15) est un capteur sans fil implantable (15).

4. Système selon l'une quelconque des revendications 1 à 3, dans lequel l'au moins un processeur (11) est configuré, lors de l'exécution des instructions de programme, pour déterminer le profil de réponse diurétique comprenant au moins l'une parmi des informations identifiant le patient comme un patient non réactif, des informations sur un traitement thérapeutique avancé pour le patient, des informations sur une résistance aux diurétiques identifiée, et des combinaisons de celles-ci.

5. Système selon l'une quelconque des revendications 1 à 4, dans lequel l'au moins un processeur (11) est configuré, lors de l'exécution des instructions de programme, pour
enregistrer, dans la base de données (14), des premières mesures de PAP obtenues auprès d'une population de patients multiples à un instant respectif t₁, dans lequel l'instant respectif t₁ est antérieur à la prise par les patients de l'au moins un médicament ;
enregistrer, dans la base de données (14), des secondes mesures de PAP obtenues auprès de la population au temps respectif t₂, dans lequel le temps respectif t₂ est postérieur au temps respectif t₁ et après que les patients ont pris l'au moins un médicament ; et
déterminer un profil de réponse diurétique de la population sur la base des première et seconde mesures de PAP.
